Europäisches Patentamt

European Patent Office

Office européen des brevets

(19)

(11) Veröffentlichungsnummer : **0 068 005**
**B1**

(12) # EUROPÄISCHE PATENTSCHRIFT

(45) Veröffentlichungstag der Patentschrift :
24.04.85

(21) Anmeldenummer : 82900185.8

(22) Anmeldetag : 21.12.81

(86) Internationale Anmeldenummer :
PCT/DE 81/00232

(87) Internationale Veröffentlichungsnummer :
WO/8202336 (22.07.82 Gazette 82/18)

(51) Int. Cl.⁴ : **A 61 F 13/20**

(54) **EINRICHTUNG ZUM ZUFÜHREN EINES TAMPON-ROHLINGS ZU EINER TAMPONPRESSE.**

(30) Priorität : 31.12.80 DE 3049581

(43) Veröffentlichungstag der Anmeldung :
05.01.83 Patentblatt 83/01

(45) Bekanntmachung des Hinweises auf die Patenterteilung : 24.04.85 Patentblatt 85/17

(84) Benannte Vertragsstaaten :
BE FR GB LU NL SE

(56) Entgegenhaltungen :
DE-C- 915 382
FR-A- 2 131 579
FR-A- 2 204 393
US-A- 2 152 230

(73) Patentinhaber : JOHNSON & JOHNSON GmbH
Kaiserswerther Strasse 270
D-4000 Düsseldorf 30 (DE)

(72) Erfinder : FRIESE, Axel
Weststrasse 68
D-5600 Wuppertal (DE)

(74) Vertreter : Groening, Hans Wilhelm, Dipl.-Ing.
Siebertstrasse 4 Postfach 860 340
D-8000 München 86 (DE)

## Beschreibung

Die Erfindung betrifft eine Einrichtung zum Zuführen eines aus saugfähigem Material, insbesondere Fasermaterial, bestehenden Rohlings für die Herstellung von Tampons, insbesondere für die Frauenhygiene, zu einer Tamponpresse gemäß dem Oberbegriff des Patentanspruchs 1.

Ausgangsmaterial zur Herstellung derartiger Tampons ist üblicherweise ein aus einer Krempelmaschine kommendes Wattevlies, das in kleine, kurze Bahnen zerteilt und zunächst zu losen Wickeln aufgerollt wird. Diese Wickel, die etwa die Länge der fertigen Tampons aufweisen, werden anschließend auf einen kleineren Durchmesser radial zusammengepreßt und gegebenenfalls noch einer axialen Pressung unterworfen. Je nach Bedarf können die Wattevliesabschnitte bereits vor dem Aufwickeln mit einem Rückholband versehen werden.

Eine Einrichtung der eingangs erwähnten Gattung ist aus der DE-PS 21 14 530 bekannt. Die der Überführungsvorrichtung für den Tampon-Rohling zugeordnete Auflagefläche besteht aus einer auf- und abbewegbaren Formbacke, mittels welcher der Rohling in die Stellung zur Überführung in die Tamponpresse angehoben und dabei vorgeformt wird, ehe der Rohling mittels eines Dornes in die Tamponpresse eingeschoben und nach dem Pressen zu einem Tampon mittels eines Ausstoßdorns nach der anderen Seite der Presse ausgestoßen wird.

Aus der DE-PS 22 53 180 ist eine Einrichtung zum Anbringen des freien Endes eines Rückholbandes an einem Tampon-Wattewickel bekannt, bei der vor Beginn des Aufwickelns des Faservlies-Streifens das freie Ende des Rückholbandes in die sich von dem Vliesstreifen weg erstreckende Lage angesaugt wird und die Saugwirkung während des Wickelvorgangs aufrechterhalten bleibt. Das Rückholband wird aus seiner Halterung beim Weitertransport des Wickels zu einer Rückholband-Anlegestation herausgezogen, in der das Rückholband-Anlegestation herausgezogen, in der das Rückholband in einen Zylinder angesaugt und danach an die Stirnseite des Wickels in Wirrlage angedrückt wird. Die Einrichtung besteht aus einem Zylindergehäuse, dessen Zylinderöffnung den zylindrischen Rohlingen jeweils etwa koaxial gegenüberliegt und einen Kolbenschieber aufnimmt, der mit Durchbrechungen versehen ist, wobei der Zylinder an eine Saugluftquelle anschließbar ist. Auf der dem Zylindergehäuse abgekehrten Seite des Wickels ist für diesen eine Anschlagfläche vorgesehen.

Gemäß der DE-PS 915 382 wird ein Rückholband in eine senkrechte Lage zu einem durch einen Schlitz eines Wickeldorns geführten Wattevlies gebracht und durch zwei Schieber über den Wickeldorn gestreift, wobei nach dem Wickeln des Vlieses zu einem Tampon-Rohling die überstehenden und um den Dorn gewickelten Enden des Rückholbandes von einem Abstreifer gegen das Ende des Rohlings gedrückt werden.

Aus der US-A-2 152 230 ist es bekannt, mit einem Rückholband versehene Rohlinge aufeinanderfolgend mittels eines endlosen Bandförderers einer Tamponpresse zuzuführen und in dieser radial zu pressen. Nach dem Pressen fallen die Tampons auf ein zweites Förderband, das sie zur Packstation überführt. Die Presse besteht aus mehreren auf einem Kreis in gleichen Abständen parallel zueinander drehbar angeordneten Kurbelwellen, die mit hierzu parallelen Kurbelzapfen versehen sind, an denen auf Zug elastisch reagierende Hebel angelenkt sind, deren anderes Ende mit dem langen Hebelarm von Preßhebeln gelenkig verbunden ist, die um feststehende zu den Kurbelwellen parallele Zapfen schwenkbar sind. Der kurze Hebelarm dieser Hebel ist als Preßbacke ausgebildet, die bei Drehung der Kurbelwellen eine hin- und hergehende Bewegung ausführt. Die Preßkräfte dieser Arme zerfallen in eine Drehkomponente und eine radiale Komponente in bezug auf die Mittelachse der Presse. Die Drehkomponenten der Preßbacken üben jedoch keine Preßwirkung auf einen zwischen den Preßbacken befindlichen Tampon aus, weil dieser durch die Backen frei getragen wird und sich aufgrund der von den Preßbacken ausgehenden Drehkomponenten dreht. Infolgedessen wirkt sich im Sinne einer Pressung des Tampons nur die radiale Kraftkomponente bei der Bewegung der Preßbacken aus. Daher wird eine Pressung des Tampons erreicht, welche dieselbe ist wie diejenige, die durch mehrere Preßbacken erzielt würde, welche radial nach innen zu der gemeinsamen Hauptachse hin bewegt werden und daher keine Drehkomponente hervorrufen. Die Innenseiten der kurzen Hebelarme sind konkav ausgebildet, um eine etwas größere Presseöffnung zu erreichen. In der Preßstellung der Backen berühren jedoch diese konkaven Flächen nicht länger den Tampon, vielmehr liegen an diesem dann nur noch die Spitzen der kurzen Hebelarme am Tampon an.

Demgegenüber liegt der Erfindung die Aufgabe zugrunde, auf kleinstem Raum unter Verminderung der Hubwege der beweglichen Maschinenteile die Einrichtung zum Zuführen von Tampon-Rohlingen zu einer Tamponpresse so zu verbessern, daß der Tampon nach der Seite, von der aus er in die Presse zugeführt wurde, unter einwandfreier Führung durch die Überführungsvorrichtung auch wieder ausgestoßen und dabei gegebenenfalls noch einer axialen Pressung unterworfen werden kann.

Die Erfindung löst diese Aufgabe durch die kennzeichnenden Merkmale des Patentanspruchs 1.

Hierdurch wird erreicht, daß nur eine einzige Überführungsvorrichtung für die Überführung des Rohlings in die Presse und die Aufnahme des Tampons aus der Presse erforderlich ist. Durch die zur zentralen Achse der Einrichtung zentrierte gemeinsame Bewegung der Trägerbacken zwischen der ersten Stellung für die Aufnahme des Rohlings und der zweiten Stellung für die Aufnahme des Tampons werden die zu überwindenden Massenträgheitskräfte erheblich reduziert, so daß der Betrieb der Überführungsvorrichtung an den Takt der Presse ohne Schwierigkeiten angepaßt werden kann. Hin- und hergehende Massen sind auf ein Minimum reduziert, wobei die kurzen Hubwege des Stößels eine hohe Produktionsleistung der Presse gewährleisten. Die von den Trägerbacken gebildete geschlossene Wandung der Durchtrittsöffnung sowie eine mindestens der Länge des Rohlings entsprechende Breite derselben in Richtung der zentralen Achse der Einrichtung gewährleistet eine einwandfreie Führung des Rohlings bzw. Tampons ungeachtet der an den Querschnitt desselben jeweils angepaßten Durchtrittsöffnung der Trägerbacken in der ersten bzw. zweiten Stellung. Schließlich wird durch die Anordnung des der Überführungsvorrichtung vorgeschalteten Stößels und des Austoßdorns auf der der Überführungsvorrichtung abgekehrten Seite der Tampon-Presse in Abhängigkeit von dem Hubweg des Ausstoßdorns auch eine axiale Pressung des Tampons möglich, die somit beim Ausstoß des Tampons aus der Presse während einer Arbeitsbewegung des Ausstoßdorns stattfindet.

Sind die Tampon-Rohlinge, wie oben beschrieben, bereits mit einem Rückholband versehen, bietet das Merkmal des Patentanspruchs 2 den besonderen Vorteil, daß das Rückholband nach dem Einschieben des Rohlings in die Presse durch den Stößel von den die Durchtrittsöffnung bildenden Trägerbacken gehalten wird und infolgedessen mit Sicherheit vor einer Einwirkung durch die Tamponpresse geschützt ist. Da der Ausstoßdorn den Tampon nach seiner Pressung in die Durchtrittsöffnung der Trägerbacken in ihrer zweiten, für den Tampon vorgesehenen Stellung mit entsprechend kleinerem Querschnitt ausstößt und bis zur Anlage an dem die Durchtrittsöffnung verschließenden Stößel verschiebt, wird auf diese Weise gleichzeitig das sich zwischen den Tampon und dem Stößel in der von den Trägerbacken gebildeten Durchtrittsöffnungen erstreckende Rückholband automatisch im wesentlichen spiralförmig zusammengeschoben und gegen das Rückholende des Tampons unter gleichzeitiger Verhakung mit den Fasern des Tampons angepreßt. Infolgedessen kann der Tampon mit dem sicher an seinem Ende anhaftenden Rückholband der Verpackungsstation zugeführt werden. Da das Rückholband das Ende des Tampons überdeckt, kann die Benutzerin beim Öffnen der Tamponverpackung das Rückholband leicht von dem Tampon lösen, bevor dieser in die Körperhöhle eingeführt wird.

Das Merkmal des Anspruchs 3 gewährleistet ein einwandfreies Zuführen des Rohlings in die Tamponpresse, wobei infolge der Reibung zwischen dem Rückholband und der geschlossenen Wandung der von den Trägerbacken gebildeten Durchtrittsöffnung in der Überführungsvorrichtung für den Rohling das Rückholband in einer zur zentralen Achse parallelen Richtung längs erstreckt wird, wobei genügend Zwischenraum zwischen dem Stößel und der Wandung der Durchtrittsöffnung vorhanden ist.

Anspruch 4 ermöglicht eine ebenso einfache wie wirksame Anordnung der Trägerbacken unter optimalen kinematischen Bedingungen.

Die Merkmale des Anspruchs 5 ermöglichen die stets geschlossene Wandung der von den Trägerbacken gebildeten Durchtrittsöffnung, gleichgültig, in welcher Schwenklage sich die Trägerbacken befinden.

Die Merkmale des Anspruchs 6 stellen sicher, daß die Trägerbacken mit ihren über die Berührungslinie mit den Spitzkanten der benachbarten Trägerbacken hinausreichenden konkaven Flächenabschnitten in der zweiten Stellung zur Aufnahme des Tampons eine kreisförmige Öffnung bilden, deren Durchmesser an den Durchmesser des Tampons angepaßt ist, so daß dieser in radialer Richtung seine Form beibehält, wenn er in axialer Richtung zumindest einem axialen Druck unterworfen wird, der zum Andrücken des Rückholbandes an das Rückziehende des Tampons ausreicht.

In der Zeichnung ist die Erfindung beispielsweise und schematisch veranschaulicht. Es zeigen:

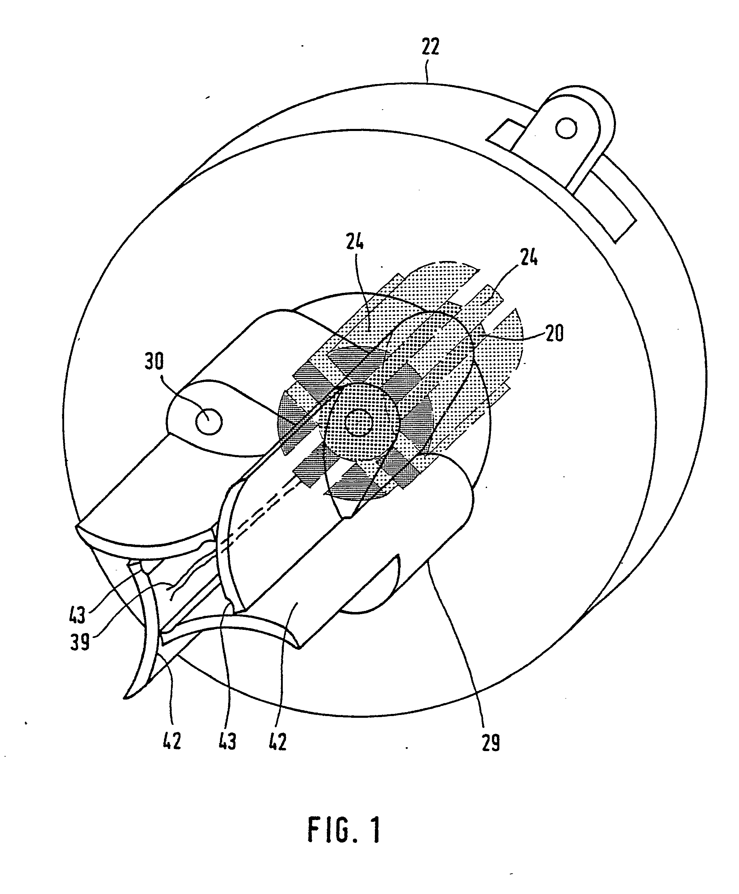

Figur 1 eine Einrichtung mit einer Überführungsvorrichtung, die einer Tamponpresse vorgeschaltet ist, in schaubildlicher Darstellung,

Figur 2 die Überführungsvorrichtung mit vier Trägerbacken in der ersten Stellung zur Aufnahme eines Rohlings in Ansicht,

Figur 3 einen Schnitt nach Linie III-III in Fig. 2,

Figur 4 die Überführungsvorrichtung nach Fig. 2 mit den Trägerbacken in der zweiten Stellung zur Aufnahme eines Tampons,

Figur 5 einen Schnitt nach Linie V-V in Fig. 4,

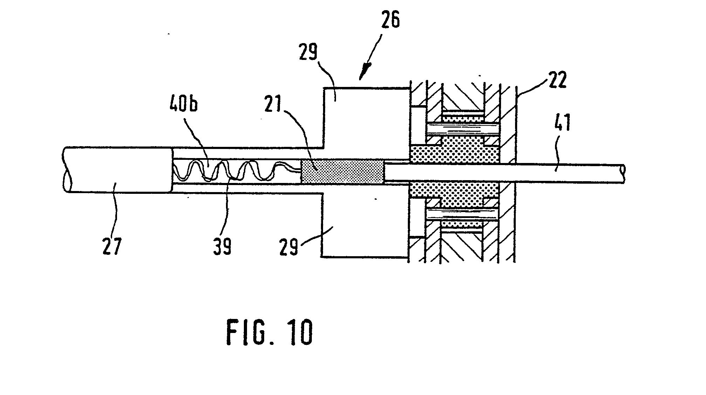

Figuren 6 bis 10 Längsschnitte der gesamten Einrichtung, die verschiedene Arbeitsphasen veranschaulichen.

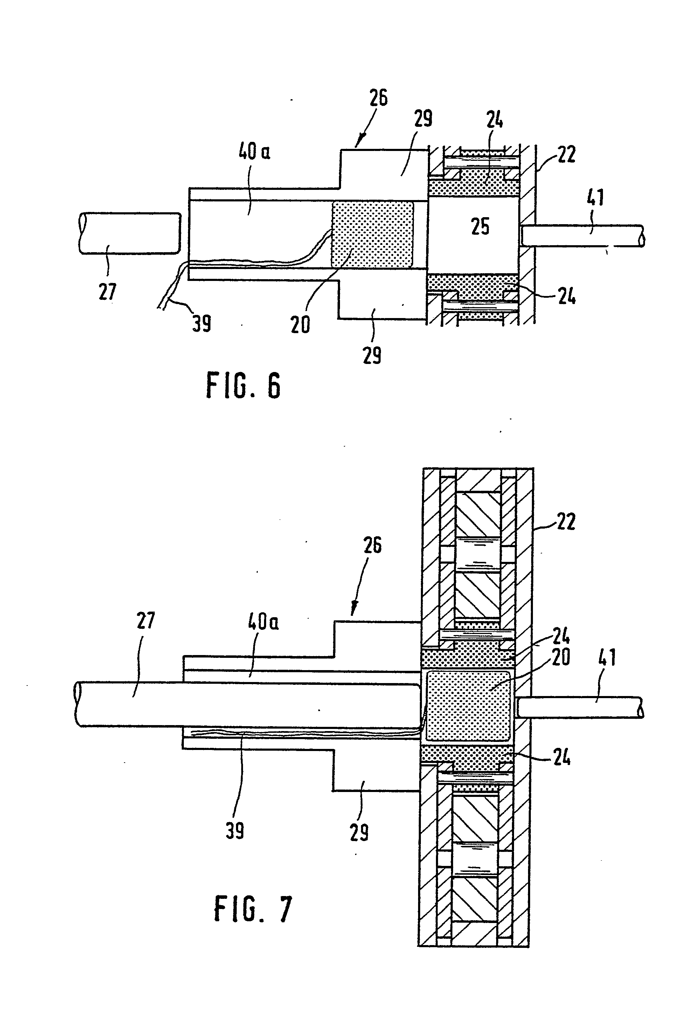

In den Figuren ist eine Einrichtung zum Zuführen eines aus saugfähigem Material, insbesondere Fasermaterial bestehenden Rohlings 20 für die Herstellung von Tampons 21, insbesondere für die Frauenhygiene, zu einer Tamponpresse 22, dargestellt, deren in einer Querebene zu einer zentralen Achse 23 der Einrichtung radial bewegbaren Preßbacken 24 eine durchgehende Pressenöffnung 25 bilden. Der Tamponpresse ist eine Überführungsvorrichtung 26 für den Rohling 20 vorgeschaltet, vor der wiederum ein Stößel 27 in Richtung der zentralen Achse 23 bis vor die Pressenöffnung 25 axial bewegbar ist (Fig. 7).

Die Überführungsvorrichtung 26 besteht aus einem Grundkörper 44, in dem ein Koppelring 28 drehbar gelagert ist. An innerhalb des Koppelrings 28 liegenden Teilen des Grundkörpers 44 sind vier Trägerbacken 29 um Drehpunkte 30 schwenkbar gelagert, die in gleichen Winkelabständen auf einem Kreis liegen, der konzentrisch zur zentralen Achse 23 bzw. dem Koppelring 28 angeordnet ist. Die Drehpunkte bestehen aus Achsen, die parallel zur zentralen Achse 23 verlaufen, d. h. die Trägerbacken 29 sind in einer Querebene zur zentralen Achse 23 schwenkbar. Im Bereich der freien, im wesentlichen nach innen gerichteten, als Spitzkanten 31 ausgebildeten Enden der Trägerbacken 29 sind diese über Bolzen 32 an Hebeln 33 angelenkt, deren anderes Ende über Lagerbolzen 34 mit dem Koppelring 28 gelenkig verbunden ist. Die Lagerbolzen 34 befinden sich wiederum auf einem gemeinsamen Kreis in voneinander gleichen Umfangsabständen, wobei der Kreismittelpunkt die zentrale Achse 23 ist. Die Hebel 33 sind jeweils gleich lang bemessen. Ebenso sind die Abstände der Bolzen 32 von der Mitte der Drehpunkte 30 der zugeordneten Trägerbacken 29 jeweils gleich. Eine Schubstange 35 ist mittels eines Bolzens 36 an einem Lagerauge 37 des Koppelrings 28 angelenkt, der in einer kreisförmigen Nut 38 im Grundkörper 44 gelagert ist und mittels einer nicht gezeigten, an der Schubstange 35 angreifenden Antriebsvorrichtung hin- und herdrehbar ist.

Die Trägerbacken 29 sind in Richtung der zentralen Achse 23 größer als die freie Länge eines Rückholbandes 39 des Rohlings 20 bemessen, so daß dieser vollständig von den Trägerbacken 29 aufgenommen wird, wenn der Rohling 20 bzw. Tampon 21 sich in der Tamponpresse 22 befindet. Selbstverständlich könnte die Breite der Trägerbacken 29 aber auch nur der Länge des Rohlings 20 etwa angemessen sein, wenn Rohlinge 20 ohne Rückholband 39 bearbeitet werden sollen.

Wie aus den Fig. 6 und 7 hervorgeht, ist der Durchmesser der Stirnfläche des Stößels 27 kleiner als die Durchtrittsöffnung 40a der Trägerbacken 29 in ihrer ersten Stellung für den Rohling 20 bemessen. Infolgedessen kann, wie Fig. 7 zeigt, der Rohling 20 durch den Stößel 27 einwandfrei in die Tamponpresse 22 überführt werden, wobei das Rückholband 39 infolge des ausreichenden Platzes ungehindert in den Zwischenraum zwischen Stößel 27 und der geschlossenen Wandung der Durchtrittsöffnung 40a nachgezogen und dort so lange gehalten werden kann, bis nach dem Pressen der Tampon 21 ausgestoßen wird.

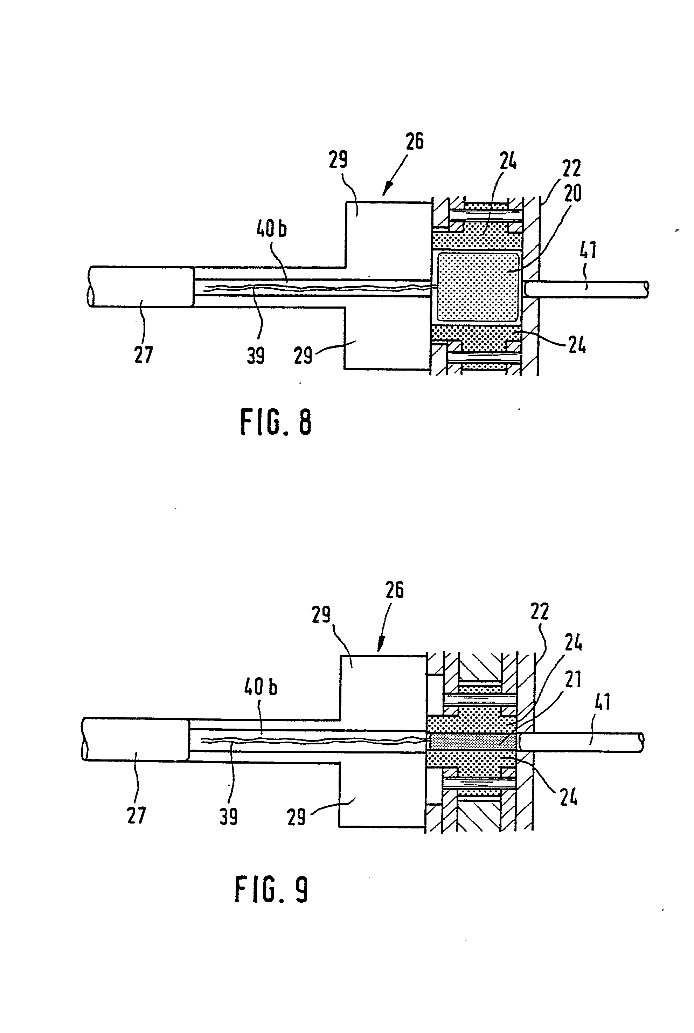

Fig. 8 und 9 zeigen, daß die Stirnfläche des Stößels 27 jedoch so groß bemessen ist, daß die Durchtrittsöffnung 40b in der zweiten Stellung der Trägerbacken 29 für die Aufnahme des Tampons 21 an dem der Tamponpresse 22 abgekehrten Ende der Trägerbacken 29 anliegt und die Durchtrittsöffnung 40b verschließt.

Ein Ausstoßdorn 41 ist auf der der Überführungsvorrichtung 26 abgekehrten Seite der Tamponpresse 22 koaxial zur zentralen Achse 23 hin- und herbewegbar gelagert und dient zum Ausstoßen des Tampons 21 aus der Tamponpresse 22 in die Überführungsvorrichtung 26 und gegen den Stößel 27, wobei das Rückholband 39 im wesentlichen Spiralform annimmt und schließlich unter Verhaken mit den Fasern des Tampons 21 an dessen Rückziehende angedrückt wird.

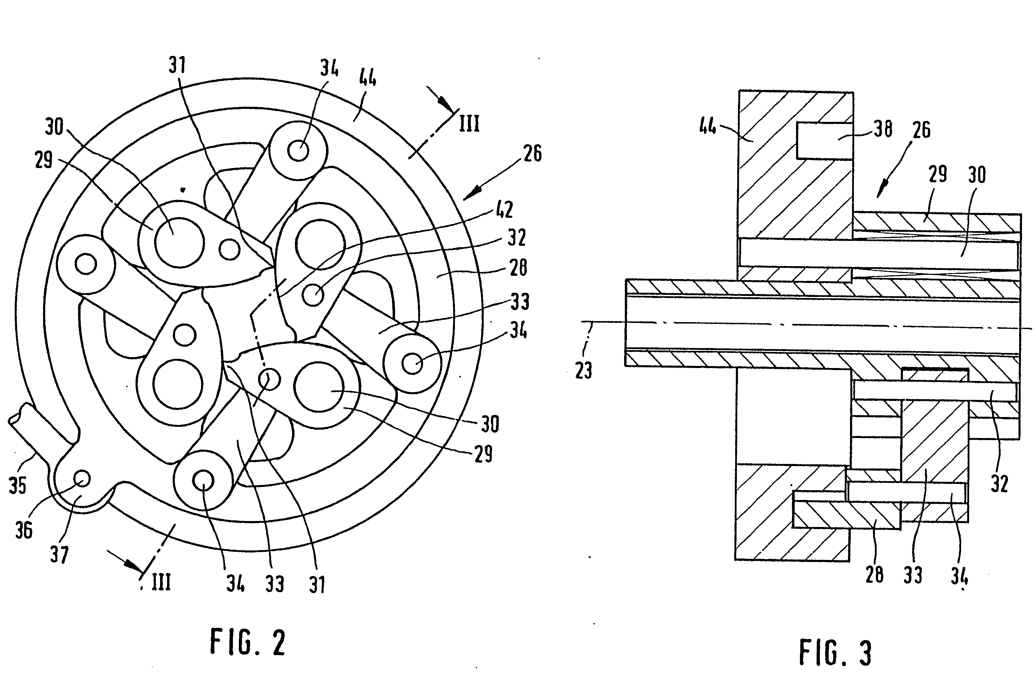

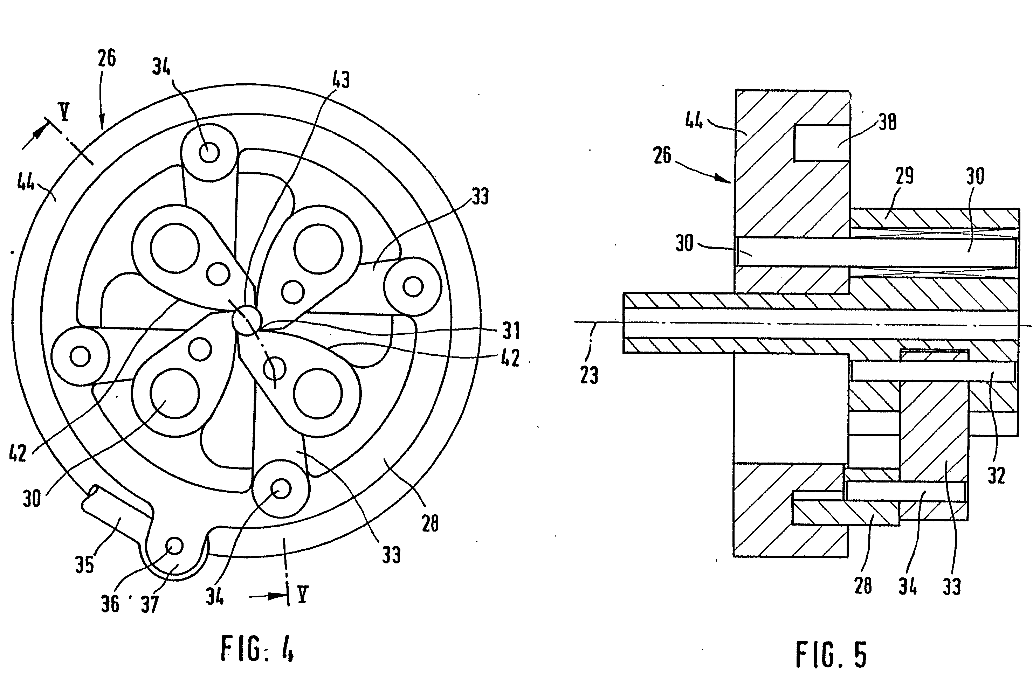

Wie die Fig. 2 und 4 zeigen, bilden die Trägerbacken 29 eine stets geschlossene Wandung für die Durchtrittsöffnung 40a bzw. 40b, was darauf zurückzuführen ist, daß die Spitzkanten 31 an den freien Enden der Trägerbacken 29 jeweils an der ihnen zugekehrten Flanke 42 einer benachbarten Trägerbacke 29 gleitend anliegen und bei der synchronen Schwenkbewegung der Trägerbacken 29 einen der konvexen Kurvenform der Flanke 42 der benachbarten Trägerbacke 29 entsprechenden Hüllkreis beschreiben. Die Spitzkanten 31 der Trägerbacken 29 liegen in ihrer zweiten, dem Querschnitt des Tampons 21 angepaßten Stellung an dem den Spitzkanten 31 zugekehrten Ende der konvexen Kurvenform der Flanke 42 der benachbarten Trägerbacke 29 an, an das sich bis zur Spitzkante 31 jeweils eine konkav gekrümmte Fläche 43 anschließt, deren Bogenlänge jeweils ein der Zahl der Trägerbacken 29 entsprechender Bruchteil des Umfangs eines Kreiszylinders mit einem dem Tampon 21 etwa entsprechenden Durchmesser ist.

Im Betrieb wird mit dem Stößel 27 der Rohling 20 durch die vieleckige Durchtrittsöffnung 40a der Überführungsvorrichtung 26 hindurch in die Tamponpresse 22 geschoben (Fig. 6, 7). Nachdem der Stößel 27 aus der Überführungsvorrichtung 26 herausgefahren ist, schließt die Überführungsvorrichtung 26 durch Drehen des Koppelringes 28 die Trägerbacken 29 zu der kreiszylindrischen Durchtrittsöffnung 40b zusammen, wobei das Rückholband 39 von den Trägerbacken 29 in Richtung auf die zentrale Achse 23 mitgenommen wird.

Im nachfolgenden Arbeitsschritt wird der Rohling 20 in der Tamponpresse 22 in üblicher Weise zum Tampon 21 gepreßt. Anschließend wird der Tampon 21 von dem Ausstoßdorn 41 aus der Tamponpresse 22 heraus in die der zweiten Stellung der Trägerbacken 29 zugeordnete kreiszylindrische Durchtrittsöffnung 40b der Überführungsvorrichtung 26 in Richtung auf den Stößel 27 geschoben, der die Durchtrittsöffnung 40b der Überführungsvorrichtung 26 verschließt. Hierbei schiebt der Tampon 21 mit seiner komprimierten Masse das Rückholband 39 vor sich her, bis es durch die Reaktionskraft des Stößels 27 gegen den Tampon 21 gepreßt wird.

Es ist somit ersichtlich, daß das Rückholband 39 nur an dem Rückziehende des Tampons 21 anliegt und vor der Inbenutzungnahme des Tampons 21 in eine Strecklage gebracht werden kann, ohne daß die Gefahr eines gleichzeitigen Ablösens oder Lockerns von Fasern des Tampons 21 gegeben ist. Ferner ist verständlich, daß anstelle der beschriebenen vier Trägerbacken 29 auch nur drei oder aber auch mehr als vier Trägerbacken 29 für die Überführungsvorrichtung 26 verwendet werden können, falls dies· erwünscht ist.

**Patentansprüche**

1. Einrichtung zum Zuführen eines aus saugfähigem Material, insbesondere Fasermaterial, bestehenden Rohlings (20) für die Herstellung von Tampons (21), insbesondere für die Frauenhygiene, zu einer Tamponpresse (22), deren in einer Querebene zu einer zentralen Achse (23) der Einrichtung radial bewegbare Preßbacken (24) eine durchgehende Pressenöffnung (25) bilden und der eine Überführungsvorrichtung (26) für den Rohling (20) vorgeschaltet ist, vor der ein Stößel (27) in Richtung der zentralen Achse (23) bis vor die Pressenöffnung (25) axial bewegbar ist, dadurch gekennzeichnet, daß mehrere Trägerbacken (29) der Überführungsvorrichtung (26) zugeordnet sind, daß die Trägerbacken (29) gemeinsam zwischen einer ersten Stellung unter Bildung einer Durchtrittsöffnung (40a) mit geschlossener Wandung für den Rohling (20) und einer zweiten Stellung unter Bildung einer Durchtrittsöffnung (40b) mit geschossener Wandung für den Tampon (21) zur zentralen Achse (23) in einem feststehenden Grundkörper (44) zentrisch bewegbar sind, daß die Breite der Trägerbacken (29) in Richtung der zentralen Achse (23) mindestens der Länge des Rohlings (20) entspricht, daß ein Ausstoßdorn (41) für den Tampon (21), dessen Durchmesser kleiner als derjenige des Tampons (21) bemessen ist, auf der den Trägerbacken (29) abgekehrten Seite der Tamponpresse (22), die aus mindestens drei gleichzeitig radial bewegbaren Preßbacken (24) besteht, bis auf einen der Länge des Tampons (21) entsprechenden Abstand gegen den Stößel (27) koaxial bewegbar ist, duch den die von Trägerbacken (29) in ihrer zweiten Stellung für den Tampon (21) gebildete Durchtrittsöffnung (40b) auf der der Tamponpresse (22) abgekehrten Seite verschließbar ist.

2. Einrichtung nach Anspruch 1, zum Zuführen von Tamponrohlingen (20) zu der Tamponpresse (22), die mit einem Rückholband (39) versehen sind, dadurch gekennzeichnet, daß die Breite der Trägerbacken (29) in Richtung der zentralen Achse (23) größer als die freie Länge des Rückholbandes (39) des Tamponrohlings (20) bemessen ist.

3. Einrichtung nach Anspruch 1 oder 2, dadurch gekennzeichnet, daß der Durchmesser der Stirnfläche des Stößels (27) kleiner als die Durchtrittsöffnung (40a) der Trägerbacken (29) in ihrer ersten Stellung für den Rohling (20) bemessen ist.

4. Einrichtung nach einem der Ansprüche 1 bis 3, dadurch gekennzeichnet, daß die Überführungsvorrichtug (26) aus vier Trägerbacken (29) besteht, welche jeweils um Drehpunkte (30) schwenkbar sind, die in dem Grundkörper (44) auf einem zur zentralen Achse (23) konzentrischen Kreis in gleichen radialen Abständen und Umfangsabständen parallel zur zentralen Achse (23) angeordnet sind, daß die freien Enden der Trägerbacken (29) an Hebeln (33) gleicher Länge angelenkt sind, deren anderes Ende an einem Koppelring (28) schwenkbar befestigt ist, dessen Durchmesser größer als derjenige des die Drehpunkte (30) der Trägerbacken (29) aufnehmenden Kreises ist und der im Grundkörper (44) konzentrisch zur zentralen Achse (23) mittels einer Stellvorrichtung (35) im Öffnungs- bzw. Schließsinn der Trägerbacken (29) hin- und herdrehbar ist.

5. Einrichtung nach einem der Ansprüche 1 bis 4, dadurch gekennzeichnet, daß die freien Enden der Trägerbacken (29) als Spitzkanten (31) ausgebildet sind, die jeweils an der ihnen zugekehrten Flanke (42) einer benachbarten Trägerbacke (29) gleitend anliegen und bei der synchronen Schwenkbewegung der Trägerbacken (29) einen der konvexen Kurvenform der Flanke (42) der benachbarten Trägerbacke (29) entsprechenden Hüllkreis beschreiben.

6. Einrichtung nach Anspruch 5, dadurch gekennzeichnet, daß die Spitzkanten (31) der Trägerbacken (29) in ihrer zweiten, dem Querschnitt des Tampons (21) angepaßkten Stellung an dem den Spitzkanten (31) zugekehrten Ende der konvexen Kurvenform der Flanke (42) der benachbarten Trägerbacke (29) anliegen, an das sich bis zur Spitzkante (31) eine konkav gekrümmte Fläche (43) anschließt, deren Bogenlänge jeweils ein der Zahl der Trägerbacken (29) entsprechender Bruchteil eines Kreiszylinders mit einem dem Tampon (21) etwa entsprechenden Durchmesser ist.

**Claims**

1. Apparatus for transmitting a blank (20) consisting of absorbent material, particularly fiber material, for the manufacture of tampons (21), particularly for feminine hygiene, to a tampon press (22), for pressing jaws (24) of which are radially movable in a plane transverse to a central axis (23) of the apparatus and form a continuous press opening (25), and on the upstream side of which there is located a transmission device (26) for the blank (20), in front of which a push-rod (27) is axially movable in the direction of the central axis up to the opening (25) of the press, characterised in that several carrier jaws (29) are associated with the transmission device (26), that the carrier jaws (29) are together movable between a first position, forming a passage opening (40a) with closed wall for the blank (20), and a second position, forming a passage opening (40b) with closed wall for the tampon (21) centrically to the central axis (23) in a stationary basic body (44), that the width of the carrier jaws (29) corresponds in the direction of the central axis (23) at least to the length of the blank (20), that an ejector mandrel (41) for the tampon (21), the diameter of which is smaller than that of the tampon (21), is coaxially movable on the side of the tampon press (22) directed away from carrier jaws (29), said tampon press consisting of at least three simultaneously radially movable pressing jaws (24), against the push-rod (27) up to a distance corresponding to the length of the tampon (21), by which push-rod (27) the passage opening (40b), formed by carrier jaws (29) in their second position, for the tampon (21) can be closed on the side facing away from the tampon press (22).

2. Apparatus according to Claim 1 for transmitting tampon blanks (26) provided with a withdrawal cord (39) to a tampon press (22), characterised in that the width of the carrier jaws (29) in the direction of the central axis (23) is greater than the free length of the withdrawal cord (39) of the tampon blank (20).

3. Apparatus according to Claim 1 or 2, characterised in that the diameter of the end face of the push-rod (27) is smaller than the passage opening (40a) of the carrier jaws (29) for the blank (20) in their first position.

4. Apparatus according to one of Claims 1 to 3, characterised in that the transmitting device (26) consists of four carrier jaws (29) each of which are pivotable about points of rotation (30) which are disposed in the basic body (44) on a circle concentric to the central axis (23) at equal radial distances and circumferential distances parallel to the central axis (23), that the free ends of the carrier jaws (29) are pivoted at levers (33) of the same length, the other ends of which are pivotably mounted on a coupling ring (28), the diameter of which is greater than that of the circle receiving the points of rotation (30) of the carrier jaws (29) and which is adapted for reversible rotary movement in the basic body (44) in the opening and closing sense of the carrier jaws (29) and concentrically to the central axis (23) by means of an adjusting device (35).

5. Apparatus according to one of Claims 1 to 4, characterised in that the free ends of the carrier jaws (29) are formed as pointed edges (31), which each slidingly engage the side of an adjacent carrier jaw (29) facing towards them and which in the synchronous pivoting movement of the carrier jaws (29) define an envelope circle corresponding to the convex curve form of the side (42) of the adjacent carrier jaw (29).

6. Apparatus according to Claim 5, characterised in that the pointed edges (31) of the carrier jaws (29) in their second position adapted to the cross-section of the tampon (21) lie against the end of the convex curve shape of the side (42) of the adjacent carrier jaw (29) which faces the pointed edges (31) and which is followed by a concavely curved surface (43) which extends up to the pointed edge (31) and the arc length of which is in each case a fraction of a circular cylinder, the diameter of which corresponds approximately to the tampon (21) said fraction corresponding to the number of the carrier jaws (29).

**Revendications**

1. Dispositif pour amener une ébauche (20), constituée en un matériau hydrophile, notamment un matériau fibreux, pour la fabrication de tampons (21), notamment pour l'hygiène féminine jusqu'à une presse à tampons (22), dont des mâchoires de pressage (24), qui sont déplaçables radialement dans un plan transversal par rapport à un axe central (23) du dispositif, forment une ouverture continue de presse (25), et en amont de laquelle est monté un dispositif de transport (26) prévu pour l'ébauche (20) et en avant duquel un poussoir (27) est déplaçable axialement, suivant la direction de l'axe central (23), jusque dans une position située directement en avant de l'ouverture (25) de la presse, caractérisé en ce que plusieurs mâchoires de soutien (29) sont associées au dispositif de transport (26), que les mâchoires de soutien (29) sont déplaçables en commun d'une manière centrée par rapport à l'axe central (23) dans un corps de base fixe (44), entre une première position, correspondant à formation d'une ouverture de passage (40a) à paroi fermée pour l'ébauche (20), et une seconde position correspondant à la formation d'une ouverture de passage (40b) à paroi fermée pour le tampon (21), que la largeur des mâchoires de soutien (29) suivant la direction de l'axe central (23) correspond au moins à la longueur de l'ébauche (20), qu'une broche (41) d'éjection du tampon (21), dont le diamètre est inférieur à celui de ce tampon (21), est déplaçable coaxialement, vers le côté, tourné vers les mâchoires de soutien (29), de la presse à tampons (22), qui est constituée par au moins trois mâchoires de pressage (24) pouvant être déplacées simultanément radialement, broche déplaçable jusqu'à venir à une distance, correspond à la longueur du tampon (21), par rapport au poussoir (27) au moyen duquel l'ouvertu.ə de passage (40b), qui est formée par les mâchoires de soutien (29) dans leur seconde position pour le tampon (21), peut être fermée sur le côté tourné à l'opposé de la presse à tampons (22).

2. Dispositif selon la revendication 1 servant à amener les ébauches de tampon (20), qui sont munies d'une bande de récupération (39), jusqu'à la presse à tampons (22), caractérisé en ce que la largeur des mâchoires de soutien (29) suivant la direction de l'axe central (23) est supérieure à la longueur libre de la bande de récupération (39) de l'ébauche de tampon (20).

3. Dispositif selon la revendication 1 ou 2, caractérisé en ce que le diamètre de la surface frontale du poussoir (27) est inférieur à l'ouverture de passage (40a) des mâchoires de soutien (29) dans leur première position pour l'ébauche (20).

4. Dispositif selon l'une des revendications 1 à 3, caractérisé en ce que le dispositif de transport (26) est constitué par quatre mâchoires de soutien (29) qui peuvent pivoter autour d'axes respectifs de rotation (30) qui sont disposés dans le corps de base (44) sur un cercle concentrique à l'axe central (23), à des distances radiales et à des distances circonférientielles égales, et ce parallèlement à cet axe central (23), que les extrémités libres des mâchoires de soutien (29) sont articulées sur des leviers (33) de longueur identique, dont l'autre extrémité est fixée de manière à pouvoir pivoter à un anneau d'accouplement (28),

dont le diamètre est supérieur à celui du cercle sur lequel se situent les axes de rotation (30) des mâchoires de soutien (29) et qui peut être entraîné selon un mouvement de rotation en va-et-vient dans le sens de l'ouverture ou de la fermeture des mâchoires de soutien (29), dans le corps de base (44), concentriquement par rapport à l'axe central (23) au moyen d'un dispositif de réglage (35).

5. Dispositif selon l'une des revendications 1 à 4, caractérisé en ce que les extrémités libres des mâchoires de soutien (29) sont réalisées sous la forme d'arêtes vives (31) qui s'appliquent en glissant respectivement contre le flanc (42) courbé, qui leur est associé, d'une mâchoire de soutien voisine (29) et décrivent lors du mouvement de pivotement synchrone des mâchoires de soutien (29), un cercle enveloppe correspondant à la forme courbe convexe du flanc (42) de la mâchoire de soutien (29) voisine.

6. Dispositif selon la revendication 5, caractérisé en ce que les arêtes vives (31) des mâchoires de soutien (29) s'appliquent, dans leur seconde position adaptée à la section transversale du tampon (21), contre l'extrémité, tournée vers les arêtes vives (31), de la forme courbe convexe du flanc (42) de la mâchoire de soutien (29) voisine, à laquelle se raccorde, jusqu'au niveau de l'arête vive (31), une surface cintrée concave (43) dont la longueur de l'arc est respectivement une fraction, correspondant au nombre des mâchoires de soutien (29), d'un cylindre droit possédant un diamètre correspondant approximativement au tampon (21).

FIG. 1

FIG. 2

FIG. 3

0 068 005

FIG. 4

FIG. 5

0 068 005

FIG. 6

FIG. 7

4

FIG. 8

FIG. 9

FIG. 10